# EUROPEAN PATENT SPECIFICATION

(11) **EP 4 006 548 B1**
(45) Date of publication and mention of the grant of the patent: **25.06.2025**
(21) Application number: 20846197.0
(22) Date of filing: 08.07.2020
(51) Int. Cl.: G01N 33/53, G01N 33/543, G01N 35/00

(54) **MULTI-BLOT TEST AUTOMATION SYSTEM**
AUTOMATISIERUNGSSYSTEM FÜR MULTIBLOT-TESTS
SYSTÈME D'AUTOMATISATION DE TESTS MULTI-TRANSFERTS

(30) Priority: 26.07.2019 KR 20190090986
(43) Date of publication of application: 01.06.2022
(73) Proprietor: Sugentech, Inc., Daejeon 34025 (KR)
(72) Inventor: YOO, Seungbum, Daejeon 35263 (KR); SONG, Moonjay, Daejeon 34209 (KR); AN, Seungsu, Sejong-si 30064 (KR); CHO, Ik, Daejeon 34092 (KR); SOHN, Mi-Jin, Daejeon 34076 (KR)
(74) Representative: Behr, Wolfgang
(86) International application number: PCT/KR2020/008903
(87) International publication number: WO 2021/020758

(56) References cited:
- WO-A1-2010/036808
- JP-A- 2014 532 869
- KR-A- 20150 086 115
- KR-A- 20150 086 115
- KR-A- 20190 058 439
- KR-B1- 101 698 497
- KR-B1- 101 698 497
- KR-B1- 101 965 299
- US-A1- 2004 241 752
- US-A1- 2005 227 370
- US-A1- 2010 140 341
- US-A1- 2010 267 049
- US-A1- 2011 111 522
- US-A1- 2013 230 844
- US-A1- 2014 227 681
- US-A1- 2014 273 189
- US-A1- 2018 128 715

## Description

### [Technical field]

The present invention relates to a multi-blot test automation system capable of testing immunoblot strips used in multiple assays.

### [Background]

As a method for detecting an analyte in a specimen, immunoblotting using an antigen-antibody reaction and Southern blotting for gene detection are used as useful test methods for multiple diagnoses. Examples of this test include allergy immunoblot tests, autoimmune immunoblot tests, and human papillomavirus (HPV) genotyping tests.

In a multi-blot test, several to tens of capture substances are immobilized on one strip, specimens are reacted, and testing is performed using detection substances. Such a strip is disclosed in documents such as Korean Patent Application Laid-Open No. 10-2019-0022871 and Korean Patent Application Laid-Open No. 10-2019-0058439. In addition, Korean Patent Laid-Open Publication No. 10-2015-0086115 discloses a system capable of automatically performing from pre-processing to analysis of a plurality of strips by fixing the strips to a device.

Here, since it is difficult for constituents such as conjugates, substrates, and the like including strips used in a multi-blot test of a related art to always show the same reactivity for each lot, it is preferable to calibrate for each lot for accuracy of the test. In the related art, there is a disadvantage in that it is not easy to respond to an occurrence of deviation for each lot because access to lot information, including correction for each lot, is not made.

In addition, when a user tests by installing two or more types of test strips, it is not only inconvenient because they have to check with the naked eyes, but also there is a possibility of accidentally installing another strip, and since an expiration date of a product should also be checked with the naked eyes, errors due to mistakes may occur.

Document US 2018/128715 A1 shows a multi-blot test automation system according to the preamble of claim 1. Further multi-blot test automation systems are known from US 2010/267049 A1, KR 101 698 497 B1 and KR 2015 0086115 A.

### [Detailed description of invention]

### [Technical problem]

An embodiment of the present invention has been devised to solve the aforementioned problems, and an object of the present invention is directed to providing a multi-blot test automation system enabling a test device to recognize unique information such as a lot number, correction value, and expiration date and matching the unique information to a test strip to perform correction for each lot.

### [Technical solution]

This object is solved by the subject matter of claim 1. Preferred embodiments of the present invention are the subject matter of the dependent claims.

In one general aspect, a multi-blot test automation system includes: a multi-block test device including a mounting module providing a plurality of strip mounting portions and an analysis module disposed above the mounting module and driven in a horizontal direction; and at least one strip mounted in the strip mounting portion of the multi-blot test device, wherein the strip includes a passage having a predetermined length and allowing a reactive solution to flow therein, one or more blot strips are disposed on a bottom surface of the passage, and a code in which lot information of the blot strip is embedded is formed on one side of the passage in a longitudinal direction.

In the multi-blot test device, a photographing unit including an image sensor is provided in the analysis module, and the analysis module is configured to perform information sensing and blot strip testing on each of a plurality of strips mounted in the mounting module.

The multi-blot test device may include a photographing unit including an image sensor, and the analysis module may perform information sensing on the strip before the blot strip testing on each of the plurality of strips mounted in the mounting module.

The strip may have an extension formed on one side of the passage, and the code may be formed in the form of a barcode or QR code on an upper surface of the extension.

The multi-blot test automation system further includes a lot information chip providing production environment information for each lot to the multi-blot test device.

The lot information chip has a body formed so as to be mountable on the strip mounting portion of the multi-blot test device, a code in which production environment information for each lot is embedded is formed on an upper surface of the body, and the analysis module of the multi-blot test device is configured to receive the production environment information for each lot from the code of the lot information chip, receive a lot number from the code of the strip, and match the production environment information of the strip.

In another general aspect, a multi-blot test method using the multi-blot test automation system of the present invention described above includes: A) updating production environment information for each lot in the multi-blot test device using the lot information chip; B) mounting a plurality of strips on a mounting module of the multi-blot test device; C) recognizing lot information formed for each strip of the plurality of strips; D) performing a blot test on the plurality of strips; and E) correcting result data for each of the plurality of strips according to individual lot information.

The lot information chip may have a body so as to be mountable on the strip mounting portion of the multi-blot test device, and a code in which production environment information for each lot is embedded may be formed on an upper surface of the body, and the step A may include A-1) mounting the lot information chip in a mounting module of the multi-blot test device; A-2) scanning a code of the lot information chip by an analysis module of the multi-blot test device; and A-3) storing the information received from the lot information chip by the multi-blot test device, and separating the lot information chip from the mounting module.

### [Effect of invention]

In the multi-blot test automation system and the multi-blot test method using the same according to the configuration of the present invention as described above, since the multi-blot test device scans a code formed in each strip to correct result data, more accurate results may be provided.

Also, in the present invention, since a strip and a code of a lot information chip are scanned using an analysis module formed in the existing device, correction may be performed for each lot, without separate equipment. Thus, according to the present invention, since the existing system is utilized, a user may more conveniently use the system and a highly efficient blot test may be performed at lower costs.

### [Brief description of drawings]

FIG. 1 is a perspective view of a multi-blot test device according to an embodiment of the present invention.
FIG. 2 is a graph showing deviations according to a lot according to an embodiment of the present invention.
FIG. 3 is a perspective view of a strip according to an embodiment of the present invention.
FIG. 4 is a perspective view of a lot information chip according to an embodiment of the present invention.
FIGS. 5 to 7 are flowcharts of a multi-blot test method according to an embodiment of the present invention.

### [Best mode]

Hereinafter, a multi-blot test automation system and a multi-blot test method using the same according to an embodiment of the present invention will be described in detail with reference to the accompanying drawings. The exemplary embodiments of the present invention to be introduced below are provided by way of example so that the idea of the present invention may be sufficiently transferred to those skilled in the art to which the present invention pertains. Accordingly, the scope of the present invention is not restricted to the following description and accompanying drawings and may be embodied in another form. In addition, throughout the specification, like reference numerals denote like components.

Here, unless indicated otherwise, the terms used in the specification including technical and scientific terms have the same meaning as those that are usually understood by those who skilled in the art to which the present invention pertains, and detailed description of the known functions and constitutions that may obscure the gist of the present invention will be omitted.

### [Multi-blot test system]

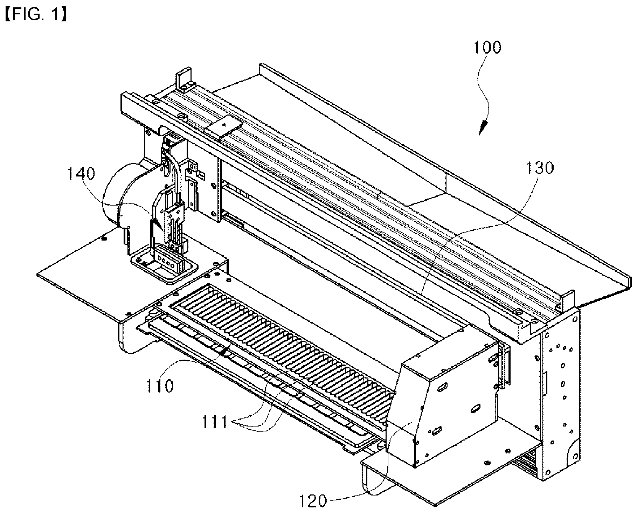

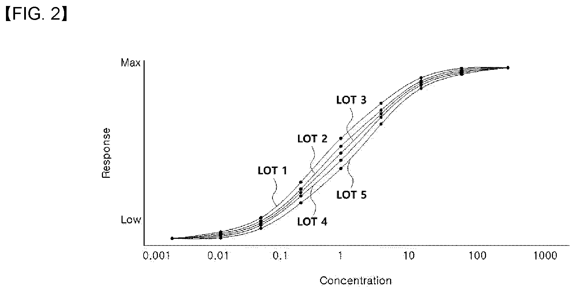

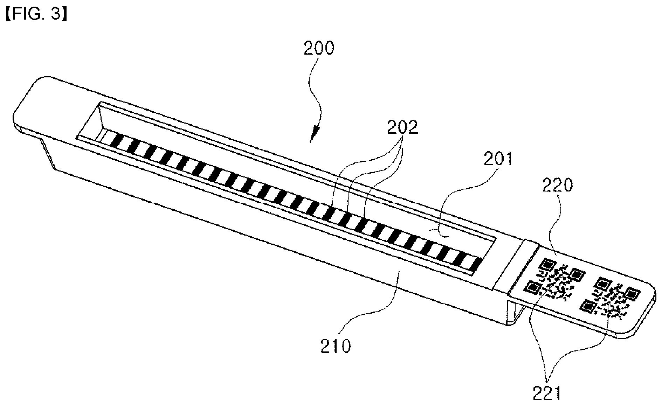

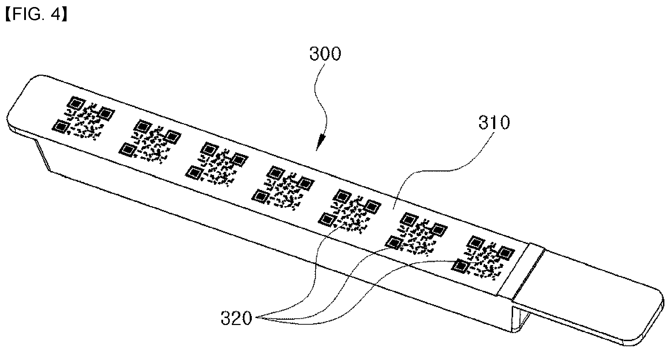

FIGS. 1 to 4 relate to an embodiment of a multi-blot test automation system according to the present invention, in which FIG. 1 is a perspective view of a multi-blot test device, FIG. 2 is a graph showing deviations according to lot, FIG. 3 is a perspective view of a strip, and FIG. 4 is a perspective view of a lot information chip.

First, referring to FIG. 1, a multi-blot test device 100 of the present invention may include a mounting module 110 in which a plurality of strip mounting portions 111 are provided and an analysis module 120 disposed above the mounting module 110 and driven in a horizontal direction. Here, the analysis module 120 may be configured to reciprocate left and right along a guide rail 130, and the analysis module 120 may include imaging equipment such as an image sensor to capture an image of a strip mounted in the strip mounting portion 111. In addition, a plurality of imaging equipment of the analysis module 120 may be provided, or a separate illuminator may be attached together, leading to various effects such as capturing a bright image of a blot strip of a strip or suppressing reflection image distortion.

In addition, the multi-blot test device 100 may further include a reagent dispensing module 140. The reagent dispensing module 140 may dispense a reagent on a plurality of strips mounted on the strip mounting portion 111. In addition, the multi-blot test device 100 may further include a device configuration for applying a specimen into each strip or washing the specimen.

Also, referring to FIG. 2, the strips inserted into the multi-blot test device 100 as described above may be identified by lot numbers according to a production environment, and strips, for which the production environment is the same but are different regarding conditions at the time of manufacturing, etc. may be managed with different lot numbers. Also, even if each lot contains a substance of the same concentration, a difference may occur in a response shown in each lot. In general, the multi-blot test device 100 is provided to quantitatively analyze a concentration through a response after a test. As described above, when the lot numbers are different, there may be a significant difference in an actual concentration even if the response is the same. In addition, a method of reducing deviations between lots through a lot system may also have a problem in that it is difficult to derive an accurate result if the deviation that has already occurred is not corrected.

Accordingly, in the present invention, as shown in FIG. 3, the multi-blot test prepared to fix at least one or more capture substances to one strip 200 is provided to correct deviations of constituents such as conjugates, substrates, etc. including the strip 200 by lot numbers. Here, data may be pre-input to the multi-blot test device 100 to correct the deviation according to the lot number, and a code 221 matching the lot number may be formed in the strip 200 of the present invention. Of course, according to the present invention, a code for directly providing production environment information or product information of the corresponding strip may be provided on the strip 200.

A structure of the strip 200 according to an embodiment of the present invention is as follows. The strip 200 may include a housing 210, and a passage 201 through which a reaction solution may flow may be formed in the housing 210 and have a predetermined length, and one or more blot strips 202 may be arranged in a lower surface of the passage 201. In addition, an extension 220 extending in the longitudinal direction of the passage 201 may be formed on one side of the housing 210, and at least one code 221 may be disposed on an upper surface of the extension 220. Here, the code 221 may be configured as a barcode or QR code and may be formed to transmit lot information of a corresponding strip to the multi-blot test device 100. In addition, when receiving the lot information of the corresponding strip, the multi-blot test device 100 may reflect it on final data using stored correction values for each lot. In addition, when there is no lot information of the corresponding strip in the storage space of the multi-blot test device 100, corresponding information may be output to an operator to receive additional information or to warn that the lot is an unauthorized lot. In addition, when it is provided to limit the lot number to be used in a test of a corresponding round, the multi-blot test device 100 may be configured to determine whether the code 221 of the strip 200 is a valid lot. Accordingly, even if the operator erroneously mounts another strip, the system may automatically determine and output that a wrong strip is mounted.

Furthermore, the code 221 of the strip 200 may further include validity period information, and validity period information of the corresponding strip scanned by the multi-blot test device 100 may be compared with the current date and it may be determined whether the corresponding strip can be used. Accordingly, the present invention may provide a more reliable test by reducing the occurrence of mistakes due to an error of the operator. In addition, a plurality of codes 221 of the strip 200 may be provided so that information such as a lot number, a result correction value, and a validity period may be arranged in each code 221.

Next, referring to FIG. 4, the present invention may further include a lot information chip 300 that provides production environment information for each lot to the multi-blot test device 100. At this time, the lot information chip 300 may be configured as a storage medium such as a USB memory, or may be configured to include a plurality of codes 320 containing data. In an embodiment of the present invention, the lot information chip 300 including the plurality of codes 320 will be described in detail.

The lot information chip 300 may contain detailed information on deviations generated for each lot number. Accordingly, the code of the strip 200 may be provided to store only the lot number, and in the present invention, the detailed information for each lot number may be preferentially received from the lot information chip 300 and a lot number may be received from the strip 200, which may then be matched.

In addition, the lot information chip 300 may be provided to recognize information through the analysis module 120 of the multi-blot test device 100, and in a state in which the lot information chip 300 is mounted in the mounting module 110, the image sensor of the analysis module 120 may scan the code 320 formed on the lot information chip 300 to receive information. Here, the lot information chip 300 may include a body 310 detachable from the strip mounting portion 111 of the mounting module 110 and a plurality of codes 320 formed on an upper surface of the body 310. Here, the code 320 may also be formed of a barcode or a QR code. In this case, in the present invention, since a space is relatively narrow in the code 221 of each strip 200, detailed information for each lot may be provided in the lot information chip 300 which is formed to provide a classification data of a wide range such as a lot number or the like and in which a blot strip or a passage is not formed, thereby enabling a more efficient data input. According to this embodiment, in the present invention, the number of codes 320 provided in the lot information chip 300 may be greater than the number of codes 221 provided in the strip 200.

### [Multi-blot test method]

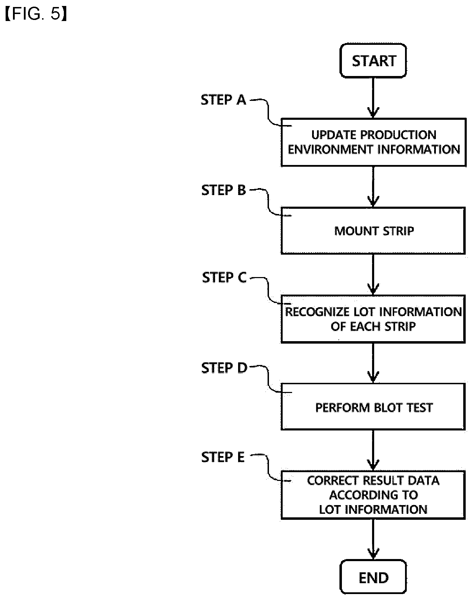

FIGS. 5 to 7 relate to an embodiment of a multi-blot test method according to the present invention, and FIGS. 5 to 7 are flowcharts of the multi-blot test method, respectively.

First, referring to FIG. 5, the multi-blot test method of the present invention may include step A of updating production environment information for each lot in the multi-blot test device 100 using the lot information chip 300, step B of mounting a plurality of strips 200 in the mounting module 110 of the multi-blot test device 100, step C of recognizing lot information formed for each of the plurality of strips 200, step D of performing a blot test on the plurality of strips 200, and step E of correcting result data according to individual lot information for each of the plurality of strips 200.

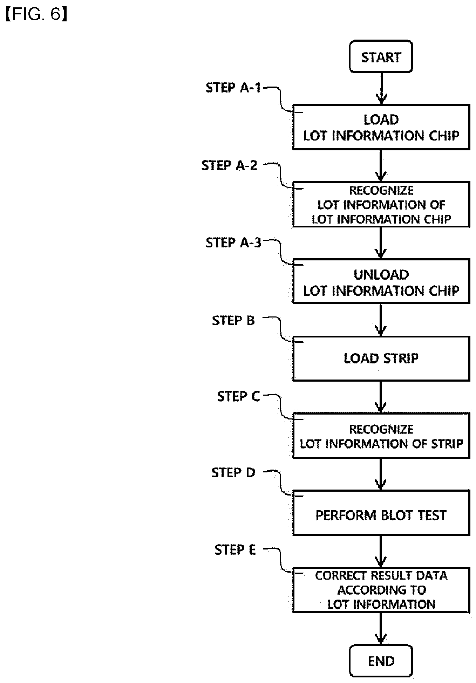

Next, referring to FIG. 6, and as described above, the lot information chip 300 may be mounted on the strip mounting portion 111 of the multi-blot test device 100, but when information is input with the code 320, step A may include step A-1 of mounting the lot information chip 300 in the mounting module 110 of the multi-blot test device 100, step A-2 of scanning of the code 320 of the lot information chip 300 by the analysis module 120 of the multi-blot test device 100, and step A-3 of storing information received from the lot information chip by the multi-blot test device 100 and separating the lot information chip 300 from the mounting module 110. Here, the lot information chip 300 may provide information on color development curves for various concentrations containing color development results of the blot strip 202 of the strip 200. Accordingly, in step **E,** regarding a blot rest result for one strip performed in step D based on the information provided from the lot information chip 300 in step A, result values may be corrected according to lot information of the one strip scanned in step C and provided.

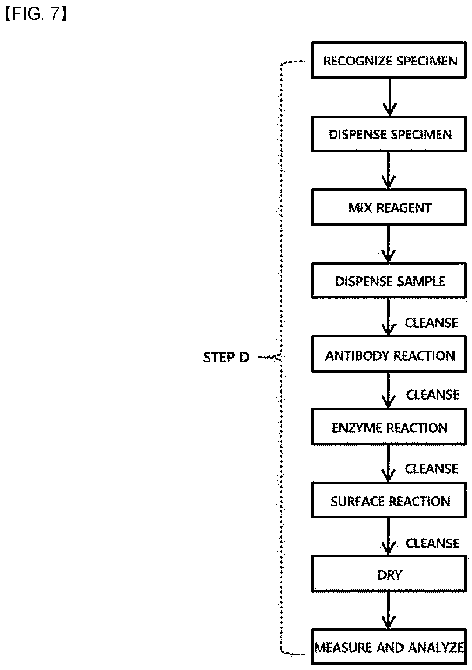

Also, step D will be additionally described as follows with reference to FIG. 7. An embodiment of step D of performing a block test on the plurality of strips 200 may include specimen recognition, specimen dispensing, reagent mixing, sample dispensing, antibody/enzyme/surface reaction test, drying, and measurement and analysis. A detailed process may vary depending on types of allergy immunoblot test, autoimmune blot test, and human papillomavirus (HPV) genotyping test, and in the case of the multi-blot test, in a state in which a plurality of capture substances are fixed to one strip, a specimen is reacted and tested using a detection material. Through this test step and test, the present invention may provide more accurate final results by recalculating preferentially analyzed result data according to a correction value for each lot of each strip.

Hereinabove, although the present invention has been described by specific matters such as detailed components, exemplary embodiments, and the accompanying drawings, they have been provided only for assisting in the entire understanding of the present invention.

## Claims

1. A multi-blot test automation system comprising:
a multi-blot test device (100) including a mounting module (110) providing a plurality of strip mounting portions (111) and an analysis module (120) disposed above the mounting module (110) and driven in a horizontal direction; and
at least one strip (200) mounted in the strip mounting portion (111) of the multi-blot test device (100),
wherein the strip includes a passage (201) having a predetermined length and allowing a reactive solution to flow therein, one or more blot strips (202) are disposed on a lower surface of the passage (201), and
a code (221) in which a lot information of the blot strip (202) is embedded is formed on one side of the passage (201) in a longitudinal direction,
a photographing unit including an image sensor provided in the analysis module (120), so that
the analysis module (120) is configured to perform information sensing and blot strip testing on each of a plurality of strips (200) mounted in the mounting module (110),
**characterized in that**
the multi-blot test automation system further comprises a lot information chip (300), wherein the lot information chip (300) has a body (310) formed so as to be mountable on the strip mounting portion (111) of the multi-blot test device (100), and a code (320) in which production environment information for each lot is embedded is formed on an upper surface of the body (310),
wherein the analysis module is configured to receive the production environment information for each lot from the code (320) of the lot information chip (300), is configured to receive a lot number from the code (221) of the strip (200), and is configured to match the production environment information of the strip (200).

2. The multi-blot test automation system of claim 1, wherein,
the strip (200) has an extension (220) formed on one side of the passage (201), and
the code (221) is formed in the form of a barcode or QR code on an upper surface of the extension (220).

3. The multi-blot test automation system of claim 1,
wherein the number of codes (320) in the lot information chip (300) is greater than the number of codes (221) in the strip (200).

4. A multi-blot test method using the multi-blot test automation system of claim 1, the multi-blot test method comprising:
A) updating production environment information for each lot in the multi-blot test device (100) using the lot information chip (300);
B) mounting a plurality of strips (200) on a mounting module (110) of the multi-blot test device (100);
C) recognizing lot information formed for each strip (200) of the plurality of strips (200);
D) performing a blot test on the plurality of strips (200); and
E) correcting result data for each of the plurality of strips (200) according to individual lot information.

5. The multi-blot test method of claim 4, wherein,
the lot information chip (300) has a body (310) so as to be mountable on the strip mounting portion (111) of the multi-blot test device (100), a code (320) in which production environment information for each lot is embedded is formed on an upper surface of the body (310), and
step A includes,
A-1) mounting the lot information chip (300) in a mounting module (110) of the multi-blot test device (100);
A-2) scanning a code (320) of the lot information chip (300) by an analysis module (120) of the multi-blot test device (100); and
A-3) storing the information received from the lot information chip (300) by the multi-blot test device (100), and separating the lot information chip (300) from the mounting module (110).

## Patentansprüche

1. Automatisierungssystem für Multi-Blot-Tests, aufweisend:
eine Multi-Blot-Testvorrichtung (100), die ein Montagemodul (110), das eine Vielzahl von Streifen-Montageabschnitten (111) bereitstellt, und ein Analysemodul (120), das oberhalb des Montagemoduls (110) angeordnet ist und in horizontaler Richtung angetrieben wird; aufweist, und
zumindest einen Streifen (200), der in dem Streifen-Montageabschnitt (111) der Multi-Blot-Testvorrichtung (100) montiert ist,
wobei der Streifen einen Kanal (201), der eine vorgegebene Länge hat und in dessen Innern eine Reaktionslösung strömen kann, aufweist, ein oder mehrere Blotstreifen (202) auf einer unteren Fläche des Kanals (201) angeordnet sind, und
einen Code (221), in den Chargeninformationen des Blotstreifens (202) eingebettet ist, auf einer Seite des Kanals (201) in einer Längsrichtung ausgebildet ist,
eine Fotografieeinheit mit einem Bildsensor, der in dem Analysemodul (120) vorgesehen ist, so dass das Analysemodul (120) derart konfiguriert ist, dass es eine Informationssensierung und einen Blotstreifen-Test an jedem einer Vielzahl von Streifen (200) durchführt, die in dem Montagemodul (110) montiert sind,
**dadurch gekennzeichnet, dass**
das Automatisierungssystem für Multi-Blot-Tests ferner einen Chargeninformationschip (300) aufweist, wobei der Chargeninformationschip (300) ein Gehäuse (310) aufweist, das so ausgebildet ist, dass es auf dem Streifen-Montageabschnitt (111) der Multi-Blot-Testvorrichtung (100) montierbar ist, und ein Code (320), in dem Produktionsumgebungsinformationen für jede Charge eingebettet sind, an einer oberen Fläche des Gehäuses (310) gebildet ist,
wobei das Analysemodul derart konfiguriert ist, dass es die Produktionsumgebungsinformationen für jede Charge von dem Code (320) des Chargeninformationschips (300) empfängt, derart konfiguriert ist, dass es eine Chargennummer aus dem Code (221) des Streifens (200) empfängt, und derart konfiguriert ist, dass es die Produktionsumgebungsinformationen des Streifens (200) abgleicht.

2. Automatisierungssystem für Multi-Blot-Tests nach Anspruch 1, wobei
der Streifen (200) einen Fortsatz (220) aufweist, der auf einer Seite des Kanals (201) ausgebildet ist, und
der Code (221) in Form eines Strichcodes oder QR-Codes auf einer oberen Fläche des Fortsatzes (220) ausgebildet ist.

3. Automatisierungssystem für Multi-Blot-Tests nach Anspruch 1,
wobei die Anzahl von Codes (320) in dem Chargeninformationschip (300) größer ist als die Anzahl von Codes (221) in dem Streifen (200).

4. Verfahren für Multi-Blot-Tests unter Verwendung des Automatisierungssystems für Multi-Blot-Tests nach Anspruch 1, wobei das Automatisierungsverfahren für Multi-Blot-Tests umfasst:
A) Aktualisieren von Produktionsumgebungsinformationen für jede Charge in der Multi-Blot-Testvorrichtung (100) unter Verwendung des Chargeninformationschips (300);
B) Montieren einer Vielzahl von Streifen (200) auf einem Montagemodul (110) der Multi-Blot-Testvorrichtung (100);
C) Erkennen von Chargeninformationen, die für jeden Streifen (200) der Vielzahl von Streifen (200) gebildet sind;
D) Durchführen eines Blot-Tests an der Vielzahl von Streifen (200); und
E) Korrigieren von Ergebnisdaten für jeden der Vielzahl von Streifen (200) gemäß individuellen Chargeninformationen.

5. Verfahren für Multi-Blot-Tests nach Anspruch 4, wobei
der Chargeninformationschip (300) ein Gehäuse (310) aufweist, so dass er auf dem Streifen-Montageabschnitt (111) der Multi-Blot-Testvorrichtung (100) montierbar ist, ein Code (320), in den Produktionsumgebungsinformationen für jede Charge eingebettet sind, an einer oberen Fläche des Gehäuses (310) gebildet ist, und
Schritt A umfasst:
A-1) Montieren des Chargeninformationschips (300) in einem Montagemodul (110) der Multi-Blot-Testvorrichtung (100);
A-2) Scannen eines Codes (320) des Chargeninformationschips (300) durch ein Analysemodul (120) der Multi-Blot-Testvorrichtung (100); und
A-3) Speichern der von dem Chargeninformationschip (300) empfangenen Informationen durch die Multi-Blot-Testvorrichtung (100) und Trennen des Chargeninformationschips (300) von dem Montagemodul (110).

## Revendications

1. Système d'automatisation de tests multi-transferts comprenant :
un dispositif de test multi-transferts (100) comportant un module de montage (110) fournissant une pluralité de parties de montage de bandes (111) et un module d'analyse (120) disposé au-dessus du module de montage (110) et entraîné dans une direction horizontale ; et
au moins une bande (200) montée dans la partie de montage de bandes (111) du dispositif de test multi-transferts (100),
dans lequel la bande comporte un passage (201) présentant une longueur prédéterminée et permettant l'écoulement d'une solution réactive en son sein, une ou plusieurs bandes de transfert (202) sont disposées sur une surface inférieure du passage (201), et
un code (221) dans lequel des informations de lot de la bande de transfert (202) sont intégrées est formé sur un côté du passage (201) dans une direction longitudinale,
une unité de photographie comportant un capteur d'image disposé dans le module d'analyse (120), de sorte que le module d'analyse (120) soit conçu pour réaliser une détection d'informations et un test de bandes de transfert sur chacune d'une pluralité de bandes (200) montées dans le module de montage (110),
**caractérisé en ce que**
le système d'automatisation de tests multi-transferts comprend en outre une puce d'informations de lot (300), dans lequel la puce d'informations de lot (300) présente un corps (310) formé pour permettre son montage sur la partie de montage de bandes (111) du dispositif de test multi-transferts (100), et un code (320) dans lequel des informations d'environnement de production pour chaque lot sont intégrées est formé sur une surface supérieure du corps (310),
dans lequel le module d'analyse est conçu pour recevoir les informations d'environnement de production pour chaque lot en provenance du code (320) de la puce d'informations de lot (300), est conçu pour recevoir un numéro de lot en provenance du code (221) de la bande (200), et est conçu pour correspondre aux informations d'environnement de production de la bande (200).

2. Système d'automatisation de tests multi-transferts selon la revendication 1, dans lequel,
la bande (200) présente une extension (220) formée sur un côté du passage (201), et
le code (221) est formé sous la forme d'un code-barres ou d'un QR code sur une surface supérieure de l'extension (220).

3. Système d'automatisation de tests multi-transferts selon la revendication 1,
dans lequel le nombre de codes (320) dans la puce d'informations de lot (300) est supérieur au nombre de codes (221) dans la bande (200).

4. Procédé de test multi-transferts utilisant le système d'automatisation de tests multi-transferts selon la revendication 1, le procédé de test multi-transferts comprenant :
A) la mise à jour d'informations d'environnement de production pour chaque lot dans le dispositif de test multi-transferts (100) utilisant la puce d'informations de lot (300) ;
B) le montage d'une pluralité de bandes (200) sur un module de montage (110) du dispositif de test multi-transferts (100) ;
C) la reconnaissance d'informations de lot formées pour chaque bande (200) de la pluralité de bandes (200) ;
D) la réalisation d'un test de transfert sur la pluralité de bandes (200) ; et
E) la correction de données de résultat pour chacune de la pluralité de bandes (200) selon des informations de lot individuelles.

5. Procédé de test multi-transferts selon la revendication 4, dans lequel,
la puce d'informations de lot (300) présente un corps (310) permettant son montage sur la partie de montage de bandes (111) du dispositif de test multi-transferts (100), un code (320), dans lequel des informations d'environnement de production pour chaque lot sont intégrées est formé sur une surface supérieure du corps (310), et
l'étape A comporte,
A-1) le montage de la puce d'informations de lot (300) dans un module de montage (110) du dispositif de test multi-transferts (100) ;
A-2) le balayage d'un code (320) de la puce d'informations de lot (300) par un module d'analyse (120) du dispositif de test multi-transferts (100) ; et
A-3) le stockage des informations reçues de la puce d'informations de lot (300) par le dispositif de test multi-transferts (100), et la séparation de la puce d'informations de lot (300) du module de montage (110).
